# DEMANDE DE BREVET EUROPEEN

(11) **EP 3 153 145 A1**
(43) Date de publication de la demande: **12.04.2017**
(21) Numéro de dépôt: 16192656.3
(22) Date de dépôt: 06.10.2016
(51) Int. Cl.: A61G 15/02, A61G 15/12, A61B 5/00, A61B 5/0408, A61G 5/10

(54) **FAUTEUIL INSTRUMENTÉ POUR L'AIDE AU DIAGNOSTIC MÉDICAL**

(30) Priorité: 06.10.2015 FR 1559516
(71) Demandeur: Stream Vision, 75009 Paris (FR)
(72) Inventeur: REBIAI, Mohamed, 95240 Cormeilles en Parisis (FR)
(74) Mandataire: Le Forestier, Eric

(57) **Abrégé**

On propose selon l'invention un fauteuil (100) pour l'aide au diagnostic médical, comprenant une unité centrale (200), un ensemble de capteurs de données physiologiques (300-1000) reliés à l'unité centrale et une interface utilisateur (117) reliée à l'unité centrale, caractérisé en ce qu'il comprend une unité d'électrocardiographie (402) comprenant au moins une électrode de main (404g, 404d) disposée sur un accoudoir du fauteuil et au moins une électrode de pied (406g, 406d) disposée sur un repose-pieds (113) faisant partie de la structure de fauteuil et apte à venir en contact avec une plante de pied, ledit repose-pieds étant ajustable en position pour faire varier sa distance par rapport à l'assise (104) du fauteuil.

## Description

### Domaine de l'invention

La présente invention concerne d'une façon générale les équipements de diagnostic médical, et plus particulièrement un équipement de type fauteuil doté d'un ensemble de moyens pour recueillir un ensemble de données physiologiques relatives à un patient notamment à des fins d'aide au diagnostic médical.

### Etat de la technique

On connaît déjà dans la littérature un certain nombre de propositions de fauteuils instrumentés à usage d'aide au diagnostic médical.

Les documents FR2898483A, US5313942A, US2011/034784A1, WO0207594A1, WO9220277A1 et WO2009034238A1 donnent un certain nombre d'exemples de tels fauteuils, dont certains incorporent une fonctionnalité d'électrocardiographie.

Toutefois, dans chacun de ces fauteuils connus, les conditions pour réaliser un électrocardiogramme de façon satisfaisante soit ne sont pas remplies, soit sont extrêmement contraignantes pour le patient.

On connaît également notamment par les documents WO 02/07594 A1, US 2009/216142 A1, US 2009/143652 A1, un équipement de diagnostic médical comprenant des agencements spécifiques pour les pieds. Toutefois ces équipements sont tous encombrants et/ou malcommodes à utiliser, en particulier si les mesures doivent impliquer le contact des pieds du patient avec des électrodes.

### Résumé de l'invention

La présente invention vise à permettre de réaliser au niveau d'un fauteuil de diagnostic médical/de santé un électrocardiogramme dans des conditions de fiabilité tout en imposant des contraintes limitées au patient.

On propose à cet effet un fauteuil pour l'aide au diagnostic médical, comprenant une unité centrale, un ensemble de capteurs de données physiologiques reliés à l'unité centrale et une interface utilisateur reliée à l'unité centrale, caractérisé en ce qu'il comprend une unité d'électrocardiographie comprenant au moins une électrode de main disposée sur un accoudoir du fauteuil et au moins une électrode de pied disposée sur un repose-pieds faisant partie de la structure de fauteuil et apte à venir en contact avec une plante de pied, ledit repose-pieds étant ajustable en position pour faire varier sa distance par rapport à l'assise du fauteuil.

L'invention comprend en outre de façon optionnelle les caractéristiques additionnelles suivantes, prises individuellement ou en toutes combinaisons que l'homme du métier appréhendera comme étant techniquement compatibles :
- le repose-pieds est sollicité par des moyens motorisés aptes à solliciter la/chaque électrode de pied en direction de la plante du/de chaque pied du patient.
- les électrodes sont amovibles tout en restant connectées à une unité d'électrocardiographie pour s'adapter à des anomalies morphologiques.
- l'interface utilisateur comprend un écran tactile monté de façon mobile sur le fauteuil, et au voisinage des électrodes d'accoudoirs des boutons de commande destinés à répliquer des commandes tactiles et situés au voisinage immédiat d'au moins une électrode de main.
- le fauteuil comprend en outre un capteur de poids apte à mesurer le poids du patient, et des moyens pour lancer un programme de diagnostic au niveau de l'unité centrale en réponse à la mesure d'un poids supérieur à un seuil.
- le fauteuil comprend en outre un capteur de poids comprenant deux platines et un ensemble de capteurs de force à compression reliant les platines l'une à l'autre, et une unité de traitement apte à recueillir et traiter les signaux des capteurs.
- l'unité de traitement précitée est apte à effectuer au moins l'un parmi un traitement additif pour détermination de poids et un traitement soustractif pour détection d'anomalie.
- le fauteuil comprend en outre des moyens mécaniques permettant sa transformation en un lit ou brancard.
- les capteurs comprennent au moins un capteur parmi un capteur de température corporelle, un capteur de tension artérielle, un capteur de rythme cardiaque, un capteur de poids, un oxymètre, un spiromètre, un capteur de déshydratation, un dispositif écho doppler.
- l'unité centrale comprend des moyens d'ordonnancement et de collecte des données fournies par les différents capteurs ou dispositifs, et des moyens pour fournir à l'interface utilisateur des indications visuelles et/ou sonores relativement à la mise en oeuvre des capteurs et pour recueillir à partir de l'interface utilisateur des instructions de déclenchement de mesure et de validation des mesures.
- le fauteuil comprend un dispositif de mesure de déshydratation par impédancemétrie relié à tout ou partie desdites électrodes et une unité de commutation des électrodes entre ledit dispositif de mesure et ladite unité d'électrocardiographie.
- l'interface utilisateur comprend un écran tactile monté sur un support à position contrôlée, et comprenant en outre des moyens pour afficher sur l'écran tactile occupant une position prédéterminée des signes pour le diagnostic de la vue.
- l'interface utilisateur comprend un transducteur acoustique, et le fauteuil comprend en outre des moyens pour diffuser des signaux acoustiques comprenant des instructions d'utilisation du fauteuil et/ou des signaux pour l'aide au diagnostic auditif et/ou des signaux pour l'aide au diagnostic de la mémoire.
- l'interface utilisateur comprend un microphone pour recueillir des signaux acoustiques comprenant des instructions vocales pour l'unité centrale et/ou des réponses vocales à des sollicitations réalisées avec lesdits signes affichés et/ou les signaux acoustiques diffusés.
- l'unité centrale comprend des moyens pour lire des informations dans une unité de mémoire portable connectée à celle-ci préalablement à l'utilisation du fauteuil, et pour écrire les données recueillies dans ladite mémoire portable.
- l'unité centrale comprend des moyens de communication par réseau avec un serveur en vue du stockage des données recueillies au niveau dudit serveur.

### Brève description des dessins

D'autres aspects, buts et avantages de la présente invention apparaîtront mieux à la lecture de la description détaillée suivante d'une forme de réalisation préférée de celle-ci, donnée à titre d'exemple non limitatif et faite en référence aux dessins annexés, sur lesquels :
La figure 1 est une vue en perspective d'un fauteuil instrumenté pour l'aide au diagnostic médical selon l'invention,
La Figure 2 est une vue de profil du fauteuil de la Figure 1,
La Figure 3 est une vue de dessus du fauteuil des Figures 1 et 2,
La Figure 4 est une vue de profil d'un détail d'une partie formant repose-pieds du fauteuil des Figures 1 à 3,
La Figure 5 est un schéma-bloc de différents modules du fauteuil selon l'invention,
La Figure 6 est une vue en plan d'un dispositif de mesure de poids équipant le fauteuil, et
La Figure 7 est une vue en coupe du dispositif de mesure de poids de la Figure 6.

### Description détaillée d'une forme de réalisation préférée

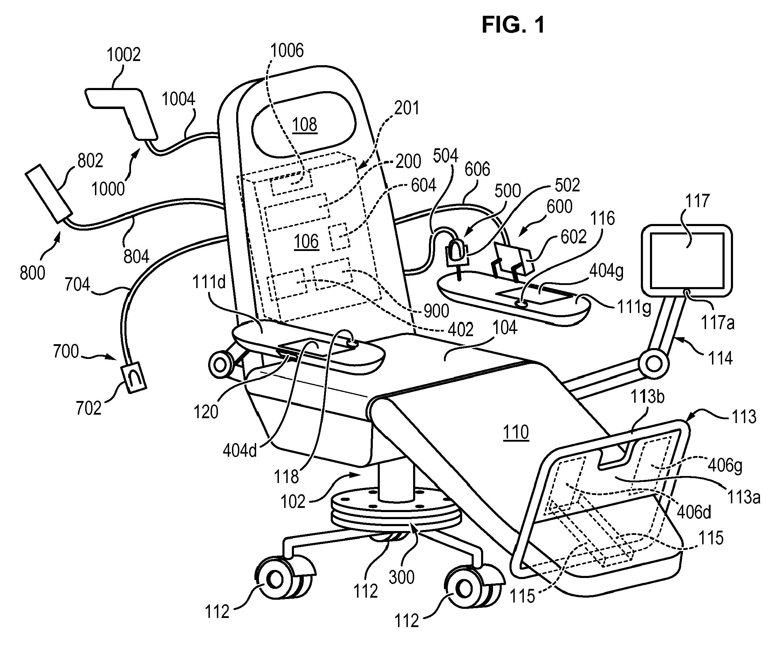

En référence aux Figures 1 à 4, on a représenté schématiquement un fauteuil 100 d'aide au diagnostic médical selon l'invention.

Il comprend un pied 102 supportant une assise 104, un dossier 106, un appuie-tête 108, un appuie-jambes 110 équipé d'un repose-pieds 113 et deux accoudoirs 111g, 111d.

De préférence la structure du fauteuil est basée sur un fauteuil médical, convertible en lit ou en brancard, du commerce. Il peut être équipé de roulettes 112 pour faciliter son déplacement.

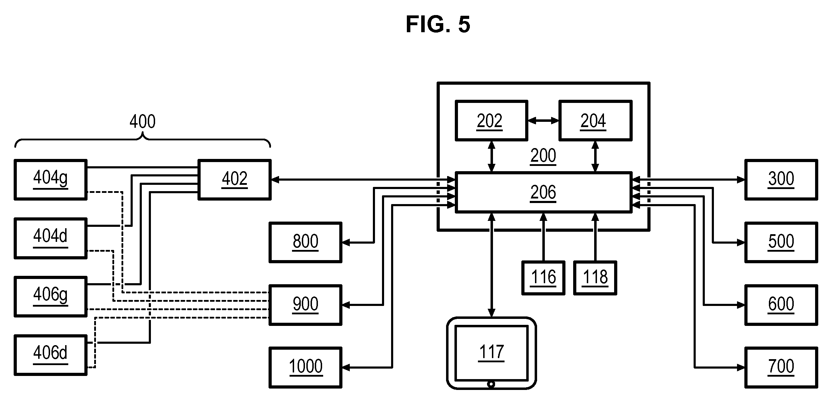

En référence à la Figure 5, une unité centrale de traitement 200, prévue par exemple dans un boîtier 201 à l'arrière du dossier 106, comprend un processeur 202, des mémoires 204 et un circuit d'entrées/sorties 206, de façon connue en soi. Cette unité centrale peut être basée par exemple sur une carte-mère d'ordinateur personnel.

Cette unité centrale 200 coopère avec une interface utilisateur. Celle-ci comprend, sur un bras articulé 114 monté sur le fauteuil, un écran tactile 117 relié à l'unité centrale 200 par les circuits d'entrée/sortie 206, ceux-ci incluant une carte vidéo appropriée.

L'interface utilisateur comprend également des boutons 116, 118 prévus sur les accoudoirs 111 g, 111 d comme on le verra en détail plus loin.

L'unité centrale comprend par ailleurs un connecteur 120, par exemple un connecteur à la norme USB prévu dans l'accoudoir 111d, permettant la connexion d'une unité de mémoire (ici une clé USB) contenant une identification du patient et des données médicales (données physiologiques, antécédents, données de diagnostics précédents, etc.).

Avantageusement, ces boutons 116, 118 permettent d'appliquer à l'unité centrale des instructions répliquant exactement des commandes tactiles sur l'écran 117, en laissant les mains sur les accoudoirs, des commandes effectuées sur l'interface tactile de l'écran 117.

Le fauteuil est équipé d'un ensemble d'équipements et capteurs spécialisés communiquant avec l'unité centrale 200 via les circuits d'entrée/sortie 206.

Ainsi un capteur de poids 300 est disposé dans le pied 102 du fauteuil de façon à effectuer une pesée du patient par mesure différentielle.

Avantageusement, ce capteur de poids permet, en détectant l'installation d'un patient sur le fauteuil, de déclencher une séquence de diagnostic comme on va le voir en détail dans la suite.

On décrira plus loin un capteur de poids particulièrement adapté à un équipement de type fauteuil ou brancard dans le domaine de la santé et notamment au fauteuil tel que décrit ici.

Le fauteuil comprend également un dispositif d'électrocardiographie 400, avec des circuits 402 placés dans le boîtier 201 de l'unité centrale et un ensemble d'électrodes, en l'espèce quatre électrodes dont deux électrodes de mains 404g, 404d et deux électrodes de pieds 406g, 406d.

Les électrodes présentent une surface de l'ordre de 50 à 200 cm2 et sont réalisées en un alliage approprié de type inox.

Les électrodes de mains 404g, 404d sont placées sur les accoudoirs 111g, 111d de telle sorte que les paumes des mains d'un patient de taille moyenne viennent s'appliquer naturellement contre lesdites électrodes.

Les électrodes de pieds 406g, 406d sont montées côte à côte sur le repose-pieds 113. Ce dernier comprend une plateforme 113a s'étendant sensiblement à angle droit par rapport au repose-jambes 110 et montée sur un support 113b apte à coulisser via une embase 113c le long d'un ou plusieurs rails ou glissières 115 montés à l'arrière de l'appuie-jambes 110 de manière à pouvoir se déplacer le long de celui-ci.

Un moteur combiné à une vis sans fin, l'ensemble étant désigné par la référence 119 et commandé par l'unité centrale 200, commande le déplacement du repose-pieds 113 vers le haut à partir d'une position basse, de manière à ce que lorsqu'un patient est installé dans le fauteuil, la plateforme 113a du repose-pieds 113 soit appliquée contre la plante des pieds du patient, et en particulier les deux électrodes des pieds 406g, 406d soient appliquées avec une fermeté appropriée contre les plantes de pieds gauche et droite, respectivement.

Avantageusement, la détection du positionnement correct du repose-pieds 113 est effectuée par détection d'un courant dans les électrodes de pieds 406g, 406d, ce courant indiquant qu'un contact correct est établi entre lesdites électrodes et les plantes des pieds.

De la sorte, il suffit au patient de s'installer dans le fauteuil et de placer ses jambes contre l'appuie-jambes 110, de placer également ses bras sur les accoudoirs 111g, 111d avec les paumes des mains en contact avec les électrodes respectives 404g, 404d, et de commander l'ensemble motorisé 119 pour amener les électrodes de pieds 406g, 406d en contact avec les plantes de pieds, pour que le dispositif d'électrocardiographie puisse être mis en oeuvre.

Alternativement, le repose-pieds 113 peut être sollicité par un ressort pour l'amener naturellement, sous l'action du ressort, dans une position telle que les électrodes de pieds 406g, 406d viennent en contact avec les plantes de pieds respectives avec la fermeté qui convient (déterminée par la force élastique).

Avantageusement, les électrodes 404g, 404d sont amovibles tout en restant connectées à unité d'électrocardiographie (par des liaisons électriques non illustrés par souci de simplification des dessins) pour s'adapter à des anomalies morphologiques (patients amputés, etc.).

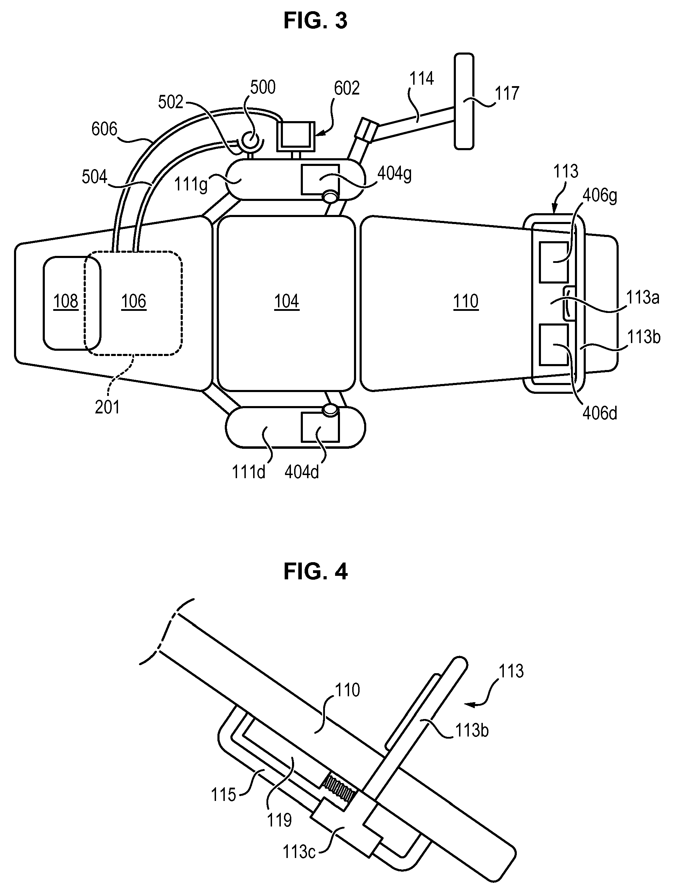

Comme le montrent en particulier les Figures 1 et 3, au voisinage des deux électrodes de mains 404g, 404d sont prévus respectivement deux boutons 116, 118, de préférence de couleurs différentes, permettant à l'utilisateur, sans que les paumes de ses mains n'aient à quitter les électrodes 404g, 404d, de solliciter l'unité centrale en réponse à des indications affichées sur l'écran tactile 117, en réplication d'une action tactile sur un bouton également affiché sur ledit écran, pour provoquer une action alors même que la réalisation d'un électrocardiogramme est en cours.

De cette manière, l'utilisateur peut piloter le déroulement des différentes opérations et mesures physiologiques alors même que ses mains (et ses pieds) sont en contact avec les électrodes respectives et donc que l'électrocardiogramme peut être effectué et enregistré.

En référence à la Figure 1, les autres dispositifs pouvant équiper le fauteuil comprennent notamment :
- un capteur de température corporelle, par exemple du type à infrarouge donc sans contact, réalisé par exemple sous la forme d'une sonde infrarouge 500 montée sur un support 502 et reliée à l'unité centrale 200 par un câble 504 ; le patient effectue la mesure sur instructions indiquées sur l'écran tactile 117 ;
- un capteur de tension artérielle 600, par exemple sous forme d'un brassard 602 relié à une pompe pneumatique 604 par un tube flexible 606, avec une connexion à l'unité centrale 200 pour la commande de gonflage/dégonflage du brassard et la mesure des pressions systolique et diastolique ;
- un capteur de rythme cardiaque et un oxymètre, ces deux capteurs étant de préférence intégrés à un dispositif d'oxymétrie de pouls 700 avec un doigtier 702 relié à l'unité centrale 200 par un câble 704, les traitements d'analyse des signaux d'origine photoélectrique recueillis au niveau du doigtier 702 sont effectués soit au niveau de l'unité centrale 200, soit au niveau d'une unité de traitement dédiée en communication avec l'unité centrale 200 ; naturellement, le rythme cardiaque peut également être déduit des signaux d'électrocardiographie ,
- un spiromètre 800, de préférence un spiromètre à turbine à interface numérique, comprenant un capteur 802 abritant les circuits de mesure du débit et relié par un câble 804 à l'unité centrale 200 ;
- un dispositif de mesure de déshydratation : de préférence, ce dispositif comprend un circuit électronique dédié 900 apte à réaliser une mesure de l'hydratation par impédancemétrie à l'aide de tout ou partie des électrodes 404g, 404d, 406g, 406d ; dans ce cas, l'unité centrale commande une unité de commutation d'électrodes, non représentée, permettant de commuter les connexions d'électrodes soit sur le circuit 900, soit sur le circuit d'électrocardiographie 402, selon la mesure à effectuer ;
- un dispositif écho-doppler 1000 permettant la prise d'images ultrasonores, ce dispositif comprenant de façon connue en soi une sonde 1002 reliée par un câble 1004 à une unité d'échographie 1006 couplée à l'unité centrale 200.

Ces différents éléments sont montés sur des supports appropriés, par exemple au niveau des accoudoirs comme illustré pour le capteur de température 500 (et son support 502) et pour le brassard de prise de tension 600), de façon à être facilement accessibles soit par le patient lui-même, soit par un personnel médical d'assistance.

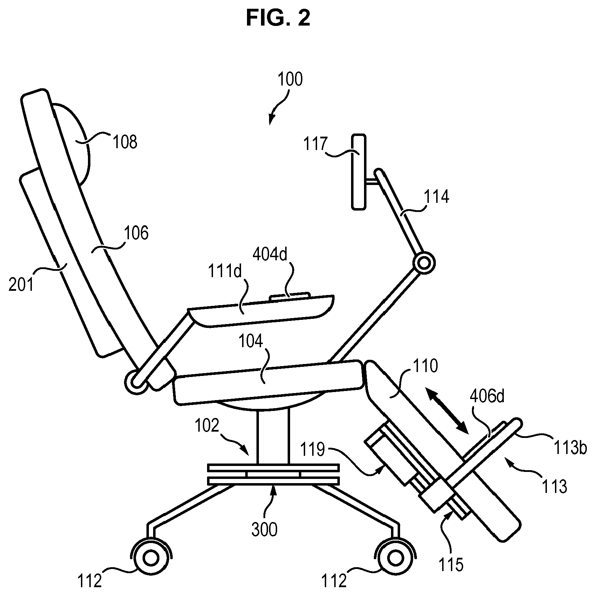

Par souci de simplification des dessins, la plupart de ces différents équipements ne sont pas représentés sur les Figures 2 à 4.

L'unité centrale, en coopération avec l'interface utilisateur, assure les fonctions principales suivantes :
- lancement du programme général d'aide au diagnostic en réponse à l'activation d'un bouton de mise en service ou, de préférence, en réponse à une détection automatique de la présence d'un patient sur le fauteuil ; comme on l'a indiqué plus haut, cette détection peut être réalisée à l'aide du capteur de poids 300, qui est interrogé périodiquement par l'unité centrale 200 en mode d'attente ; alternativement, cette détection peut être réalisée à l'aide d'un capteur de présence de technologie appropriée) ou en détectant la présence d'une partie corporelle sur une ou plusieurs des électrodes via la détection d'un micro-courant circulant dans le corps, etc. ; on notera que ce programme inclut, antérieurement au lancement de la réalisation d'un électrocardiogramme et de préférence en début de programme, la commande de l'ensemble motorisé 119 pour assurer la bonne mise en contact des électrodes de pieds 406g, 406d avec les électrodes de pieds ;
- affichage sur l'écran 117 d'instructions à destination du patient ou d'une personne assistant le patient dans l'aide au diagnostic, pour successivement effectuer les différentes mesures à l'aide des équipements décrits dans ce qui précède ; ces instructions comprennent notamment des indications pour la mise en place de l'équipement sur le patient, et la présence de différents boutons permettant de déclencher la mesure, puis passer à l'étape suivante ; ces instructions peuvent comprendre également toute indication de nature à signaler un mauvais positionnement et/ou une mauvaise utilisation de l'un des équipements du capteur ; notamment, la qualité du contact entre les mains et les pieds du patient et les électrodes respectives ;

- à mesure que les différents équipements sont utilisés, enregistrement des différentes mesures dans un dossier médical dans la mémoire locale 204 de l'unité centrale 200 et/ou sur l'unité de mémoire portable reliée au connecteur 120 et/ou, via un circuit de communication par réseau avec un serveur central, dans le serveur central en question ;
- à l'aide d'un menu spécifique accessible seulement à certaines catégories de personnel, diagnostic technique et maintenance du fauteuil et de ses différents équipements.

Le fauteuil selon l'invention peut également être doté de moyens d'aide au diagnostic visuel et/ou auditif.

Pour le diagnostic visuel, l'unité centrale peut être programmée pour afficher sur l'écran 117 des signes tels que des lettres et chiffres, en s'assurant d'une manière ou d'une autre que l'écran 117 se trouve à une distance déterminée des yeux du patient et sensiblement perpendiculairement à la direction de visée. Ceci peut être réalisé par exemple en prévoyant des moyens motorisés pour déplacer le bras 114 et l'écran jusque dans la position souhaitée sous le contrôle de l'unité centrale 200. Alternativement, ceci peut être réalisé en prévoyant des moyens de détection de la position de l'écran dans l'espace, en déplaçant l'écran manuellement et en affichant sur l'écran une indication lorsque celui-ci a été amené dans la position correcte.

Un microphone 117a par exemple intégré à l'écran permet alors d'enregistrer les lettres et chiffres lus par le patient et, par des fonctionnalités connues en elles-mêmes de reconnaissance vocale, de déterminer si le patient arrive à lire les signes à mesures qu'ils sont affichés dans des tailles de plus en plus petites.

Par ailleurs, une aide au diagnostic auditif et/ou une aide au diagnostic de mémoire peuvent être mises en oeuvre en diffusant des sons (signaux sonores ou phrases lues) à l'aide d'un transducteur acoustique tel qu'un haut-parleur (non représenté) et en enregistrant les réactions de l'utilisateur soit à l'aide du microphone 117a, soit à l'aide d'actions sur les boutons 116, 118, soit encore à l'aide de la fonction tactile de l'écran 117.

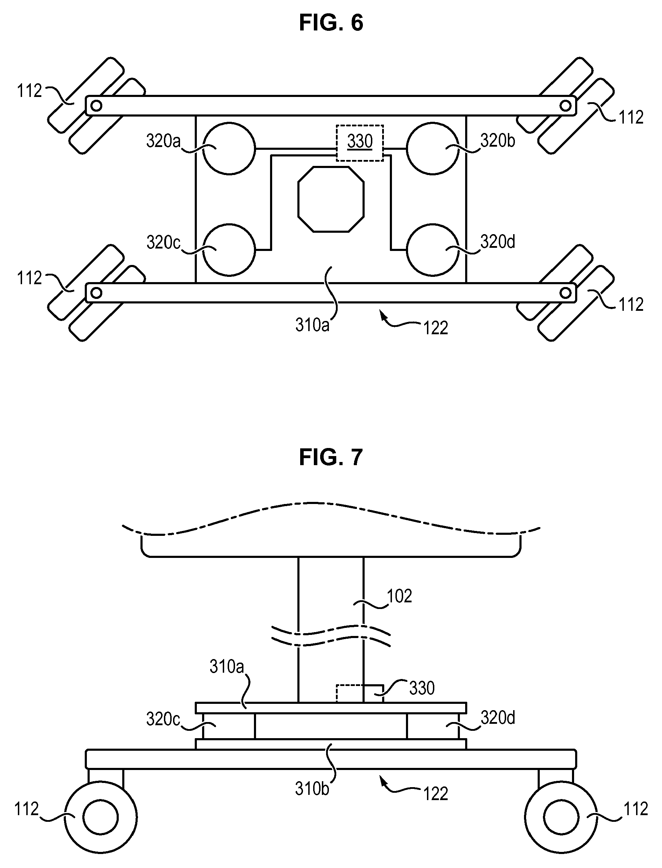

En référence maintenant aux Figures 6 et 7, on va décrire un dispositif de mesure de poids qui peut être avantageusement utilisé avec un équipement médical tel que fauteuil ou brancard, convertible ou non. En particulier, ce capteur de poids constitue une invention distincte qui peut être utilisé avec ou sans les équipements du fauteuil tel que décrit dans ce qui précède. Dans le premier cas, il constitue le capteur de poids 300 illustré sur les Figures 1, 2 et 5.

Ce capteur de poids, désigné également par la référence 300, comprend deux platines 310a, 310b, respectivement supérieure et inférieure, possédant des aménagements pour un ensemble de capteurs de poids, de préférence quatre capteurs de poids 320a, 320b, 320c, 320d, agencés de préférence aux quatre coins d'un carré ou d'un rectangle. La platine supérieure 310a est reliée rigidement à une ossature supérieure du fauteuil via un pied 102, tandis que la platine inférieure 310b est reliée rigidement à une structure de piètement 122 du fauteuil, par exemple équipée de roulettes comme illustré sur les Figures 1 et 2.

Ces capteurs sont de préférence des capteurs de force à compression à jauge de contrainte, tels que ceux commercialisés par la société Mesurex, Saint-Arnoult-en-Yvelines, France.

Les quatre capteurs sont connectés à une unité de traitement 330 dont le rôle premier est de réaliser une addition des signaux fournis par les quatre capteurs, de manière à générer un signal de poids total. Dans le cas d'une implémentation avec le fauteuil des Figures 1 à 5, le signal est délivré à l'unité centrale de traitement 200 via les circuits d'entrée/sortie 206.

Une telle configuration du dispositif de mesure de poids permet de réaliser une mesure de poids de façon fiable et sans compromettre la stabilité du fauteuil.

A cet égard, le recours à un capteur unique au niveau d'un pied principal du fauteuil donnerait lieu à des phénomènes de flexion susceptibles de nuire mécaniquement au capteur ainsi qu'à la précision des mesures.

Par ailleurs, le recours à une pluralité de capteurs permet, par examen comparatif des signaux de force délivrés par chacun des capteurs, de détecter des anomalies et/ou vérifier que le patient est bien installé dans le fauteuil.

Par exemple, en comparant les signaux de force à gauche et à droite du fauteuil, on peut vérifier que le patient est convenablement centré dans le fauteuil.

Par ailleurs, on peut prévoir que le processus d'initialisation du fauteuil décrit en référence aux Figures 1 à 5 débute dès qu'au moins un capteur de force démontre un accroissement de la force mesurée, ceci témoignant de l'instant où un patient commence à s'asseoir dans le fauteuil.

Dans le cas d'une application à des lits hospitaliers, une structure de mesure de poids telle que décrite permet non seulement de réaliser la mesure régulière du poids d'un patient, mais également de détecter (et d'alerter le personnel de soins) un éventuel déséquilibre entre les différents capteurs, révélant par exemple qu'un patient en soins en train de dormir s'approche trop dangereusement du bord du lit.

Bien entendu, l'invention n'est nullement limitée aux formes de réalisation décrites ci-dessus et représentées sur les dessins, mais l'homme du métier saura y apporter de nombreux compléments et modifications.

En particulier, le fauteuil peut être équipé pour identifier et/ou authentifier automatiquement un patient avant la réalisation des mesures (identification biométrique ou autre).

## Revendications

1. Fauteuil (100) pour l'aide au diagnostic médical, comprenant une unité centrale (200), un ensemble de capteurs de données physiologiques (300-1000) reliés à l'unité centrale et une interface utilisateur (117) reliée à l'unité centrale, **caractérisé en ce qu'**il comprend une unité d'électrocardiographie (402) comprenant au moins une électrode de main (404g, 404d) disposée sur un accoudoir du fauteuil et au moins une électrode de pied (406g, 406d) disposée sur un repose-pieds (113) faisant partie de la structure de fauteuil et apte à venir en contact avec une plante de pied, ledit repose-pieds étant ajustable en position pour faire varier sa distance par rapport à l'assise (104) du fauteuil.

2. Fauteuil selon la revendication 1, dans lequel le repose-pieds (113) est sollicité par des moyens motorisés (119) aptes à solliciter la/chaque électrode de pied en direction de la plante du/de chaque pied du patient.

3. Fauteuil selon l'une des revendications 1 et 2, dans lequel les électrodes (404g, 404d, 406g, 406d) sont amovibles tout en restant connectées à une unité d'électrocardiographie (402) pour s'adapter à des anomalies morphologiques.

4. Fauteuil selon l'une des revendications 1 à 3, dans lequel l'interface utilisateur comprend un écran tactile (117) monté de façon mobile sur le fauteuil, et un ensemble de boutons de commande (116, 118) destinés à répliquer des commandes tactiles et situés au voisinage immédiat d'au moins une électrode de main (404g, 404d).

5. Fauteuil selon l'une des revendications 1 à 4, comprenant en outre un capteur de poids (300) apte à mesurer le poids du patient, et des moyens pour lancer un programme de diagnostic au niveau de l'unité centrale en réponse à la mesure d'un poids supérieur à un seuil.

6. Fauteuil selon l'une des revendications 1 à 4, comprenant en outre un capteur de poids comprenant deux platines et un ensemble de capteurs de force à compression reliant les platines l'une à l'autre, et une unité de traitement (330) apte à recueillir et traiter les signaux des capteurs.

7. Fauteuil selon la revendication 6, dans lequel l'unité de traitement est apte à effectuer au moins l'un parmi un traitement additif pour détermination de poids et un traitement soustractif pour détection d'anomalie.

8. Fauteuil selon l'une des revendications 1 à 7, lequel comprend en outre des moyens mécaniques permettant sa transformation en un lit ou brancard.

9. Fauteuil selon l'une des revendications 1 à 8, dans lequel les capteurs comprennent au moins un capteur parmi un capteur de température corporelle (500), un capteur de tension artérielle (600), un capteur de rythme cardiaque (700), un capteur de poids (300), un oxymètre (700), un spiromètre (800), un dispositif de mesure de déshydratation (900), un dispositif écho doppler (1000).

10. Fauteuil selon la revendication 9, dans lequel l'unité centrale (200) comprend des moyens d'ordonnancement et de collecte des données fournies par les différents capteurs ou dispositifs, et des moyens pour fournir à l'interface utilisateur (117) des indications visuelles et/ou sonores relativement à la mise en oeuvre des capteurs et pour recueillir à partir de l'interface utilisateur des instructions de déclenchement de mesure et de validation des mesures.

11. Fauteuil selon l'une des revendications 1 à 10, comprenant un dispositif de mesure de déshydratation par impédancemétrie (900) relié à tout ou partie desdites électrodes (404g, 404d, 406g, 406d) et une unité de commutation des électrodes entre ledit dispositif de mesure et ladite unité d'électrocardiographie.

12. Fauteuil selon l'une des revendications 1 à 11, dans lequel l'interface utilisateur comprend un écran tactile (117) monté sur un support (114) à position contrôlée, et comprenant en outre des moyens pour afficher sur l'écran tactile occupant une position prédéterminée des signes pour le diagnostic de la vue.

13. Fauteuil selon l'une des revendications 1 à 12, dans lequel l'interface utilisateur comprend un transducteur acoustique, et le fauteuil comprend en outre des moyens pour diffuser des signaux acoustiques comprenant des instructions d'utilisation du fauteuil et/ou des signaux pour l'aide au diagnostic auditif et/ou des signaux pour l'aide au diagnostic de la mémoire.

14. Fauteuil selon l'une des revendications 12 et 13, dans lequel l'interface utilisateur comprend un microphone (117a) pour recueillir des signaux acoustiques comprenant des instructions vocales pour l'unité centrale et/ou des réponses vocales à des sollicitations réalisées avec lesdits signes affichés et/ou les signaux acoustiques diffusés.

15. Fauteuil selon l'une des revendications 1 à 14, dans lequel l'unité centrale (200) comprend des moyens pour lire des informations dans une unité de mémoire portable connectée (120) à celle-ci préalablement à l'utilisation du fauteuil, et pour écrire les données recueillies dans ladite mémoire portable.
